(19) Europäisches Patentamt
European Patent Office
Office européen des brevets

(11) Publication number : **0 500 472 A2**

# (12) EUROPEAN PATENT APPLICATION

(21) Application number : **92400462.5**

(51) Int. Cl.$^5$ : **B01D 39/14,** A61M 1/38

(22) Date of filing : **21.02.92**

(30) Priority : **22.02.91 JP 48645/91**

(43) Date of publication of application :
**26.08.92 Bulletin 92/35**

(84) Designated Contracting States :
**BE DE FR GB IT NL SE**

(71) Applicant : **TERUMO KABUSHIKI KAISHA**
**No. 44-1, Hatagaya 2-chome, Shibuya-ku**
**Tokyo 151 (JP)**

(72) Inventor : **Katsurada, Naoki, c/o Terumo Kabushiki Kaisha**
**1500 Inokuchi, Nakai-machi**
**Ashigarakami-gun, Kanagawa-ken (JP)**
Inventor : **Wakabayashi, Sobei**
**12-14, Namiyose-cho**
**Fukui-shi, Fukui-ken (JP)**
Inventor : **Hasimukai, Tomoko**
**68-24, Kamihyogo, Sakaimachi**
**Sakai-gun, Fukui-shi, Fukui-ken (JP)**

(74) Representative : **Gillard, Marie-Louise et al**
**Cabinet Beau de Loménie 55, Rue d'Amsterdam**
**F-75008 Paris (FR)**

## (54) Leukocyte-removing filter and leukocyte-removing apparatus furnished therewith.

(57) A filter for removing leukocytes and thrombocytes from a solution containing blood components, such as CRC- comprises a filter material having a surface on which zeta potential is a positive value in an electrolyte solution having a pH value of 5.0 to 7.0. In a case where the filter material originally has a negative zeta potential, the filter material to be used in the filter is pretreated so that zeta potential thereon becomes a positive value in an electrolyte solution having pH value of 5.0 to 7.0.

EP 0 500 472 A2

## BACKGROUND OF THE INVENTION

### Field of The Invention:

This invention relates generally to a leukocyte-removing filter and a leukocyte-removing apparatus furnished therewith. More specifically, the invention relates to a leukocyte-removing filter capable of removing leukocytes from a solution containing blood components while efficiently removing thrombocytes or blood platelets therefrom.

### Description of The Prior Art:

In recent years, blood transfusion has been changing from whole blood transfusion to component blood transfusion, so that a component medicine containing pure erythrocytes has been required. Generally, blood obtained from donors is fractionated into various components by centrifugation so that erythrocytic component is separated from other components in the form of a concentrated red cell, which will be hereinafter referred to as "CRC", to be transfused into patients who need erythrocytes. However, since much a CRC copiously contains other blood cells, such as leukocytes and thrombocytes, there are serious problems in that side effects tend to be caused by the components other than erythrocytes after transfusion. Particularly, side effects, such as nonhemolytic pyrogenic side effects, production of antileukocytic antibody, and graft v.s. host reaction, are often caused by leukocytes after transfusion. For that reason, leukocytes are usually removed from CRC by filtration. Furthermore, a case where transfusion is frequently repeated, it is desired to remove thrombocytes from CRC since it is required to prevent thrombocytes from producing antithrombocytic antibody.

Heretofore, in order to remove thrombocytes along with leukocytes, a filter of a small pore size (distances between fibers are small in a case where the filter comprises fibers) is used. However, when the pore size of the filter becomes small, the passing speed of blood through the filter becomes low, so that there is a disadvantage in that the filtration and removal of leukocytes and thrombocytes take a lot of time.

## SUMMARY OF THE INVENTION

It is therefore a principal object of this invention to probide a filter which can efficiently remove leukocytes together with thrombocytes by filtration or adsorption from a solution containing blood cells, such as CRC, and an apparatus furnished therewith.

In order to accomplish the aforementioned and other objects, a leukocyte-removing filter of this invention comprises a porous filter material having a surface serving as a contact surface with a processed solution, the zeta potential of the contact surface being a positive value, preferably not less than 3 mV, in an electrolyte solution of a pH value of 5,0 to 7.0. Furthermore, a leukocyte-removing apparatus of this invention contains the aforementioned leukocyte-removing filter.

According to one aspect of this invention, a filter for removing leukocytes and thrombocytes from a solution containing blood components comprises a filter material having a surface on which zeta potential is a positive value in an electrolyte solution having a pH value of 5.0 to 7.0.

According to another aspect of this invention, an apparatus for removing leukocytes and thrombocytes from a solution containing blood components comprises: a filter having a surface on which zeta potential is a positive value in an electrolyte solution having a pH value of 5.0 to 7.0; and a container for housing therein said filter, the container having an inlet for introducing the solution containing blood components into the interior thereof, and an outlet for discharging a filtrate to the outside.

## BRIEF DESCRIPTION OF THE DRAWINGS

This invention will be understood more fully from the detailed description given herebelow and from the accompanying drawings of the preferred embodiments of the invention. However, the drawings are not intended to imply limitation of the invention to a specific embodiment, but are for explanation and understanding only.

In the drawings:

Fig. 1 is a schematic view of the first preferred embodiment of an leukocyte-removing apparatus, according to this invention; and

Fig. 2 is a schematic view of the second preferred embodiment of an leukocyte-removing apparatus, according to this invention.

DESCRIPTION OF THE PREFERRED EMBODIMENT

According to this invention, a leukocyte-removing filter comprises a porous filter material having a surface on which zeta potential is a positive value in an electrolyte solution having a pH value of 5.0 to 7.0. By using such a porous filter material, it is possible to efficiently remove thrombocytes without reducing pore size of the porous filter material, so that it is possible to efficiently and quickly perform filtration and removal of leukocytes and thrombocytes.

In this specification, the term "porous filter material" means a filter material having a plurality of fine gores which establish communications between the opposite surfaces thereof. As a leukocyte-removing filter of this invention, various porous filter materials may be used, provided that they have liquid permeability and average pore size for allowing selective removal of leukocytes. There is no specific limitation to the shapes and communication state of the pores. There is also no specific limitation to thickness, material, shape, size and so forth of the filter material. The filter material may be made in various forms, such as film, sheet, membrane, plate, block, fiber and particle. The filter material may be made of either organic or inorganic material, and either a natural material, synthetic material (resin) or a semisynthetic material (resin). For example, the porous filter material of this invention may include a porous body of organic or inorganic fibers made of a natural, synthetic, semisynthetic or reproduced material; an organic or inorganic foams; an organic or inorganic porous body leaving a plurality of pores formed by elution of pore components, sintering, streching or perforating; or a porous body filled with or connected to organic or inorganic fine particles or fine pieces. For example, porous filter material made of fibers may be organic or inorganic short fibers, slibers or long fibers. The filter material may be made in various forms, such as a simple packing material of fiber, an aggregation, nonwoven fabric, and knit or woven fabric. According to this invention, in a case where the filter materials are made of fibers, they are preferably cellulose fibers, such as Egyptian cotton, or regenerated cellulose fibers, and in a case where the filter materials are porous bodies, they are preferably made of polyvinyl alcohol crows-linked with hormaldehyde or polyurethane.

In this specification, the term "zeta potential" means a value represented by voltage which corresponds to a relative potential on an interface of a solid-liquid dispersion system, i.e. a solution in which solid particles are suspended, when it is assumed that the potential in the solution is zero. Methods for measuring zeta potential are broadly divided into methods using electrophoresis method, electroendosmosis method, flow potential method and precipitation potential method. In the preferred embodiments of this invention, a method using flow potential method was used. As is well known, in this method, zeta potential is determined by measuring electrical outputs caused by mechanical energy applied to the dispersion system. Methods for measuring zeta potential using flow potential method, and systems using same are described in "Membrane Experiment Method" by Masayuki Nakagaki (Kitami Shobo Co., Ltd.), pages 93-95, JP-A-61-77752 and JP-A-63-200052. In the preferred embodiments of this invention, Shimazu Flow Potential Measuring Apparatus ZP-10B (produced by Shimazu Seisakusho Co., Ltd.) was used. By using the measured potential in Helmholts-Smoluchowski's formula as expressed by the following formula I, zeta potential was calculated.

$$\text{Zeta Potential (mV)} = (4\pi\eta/\varepsilon) \times (Es\lambda/P) \quad (1)$$

wherein $\eta$ is coefficient of kinematic viscosity of the solution, $\varepsilon$ is dielectric constant of the solution, $\lambda$ is electrical conductivity of the solution, and P is pressure applied to the solution in order to cause the solution to flow.

According to this invention, if the porous filter material to be used has a positive zeta potential in an electrolyte solution having a pH value of 5.0 to 7.0, it may be used as a material of the leukocyte-removing filter as it stands. On the other hand, if the porous filter material has a negative zeta potential in the aforementioned solution, it may be used as a material of the leukocyte-removing filter after it is treated to change its zeta potential to a positive value. Furthermore, even if the zeta potential is a positive value, it may be treated to further increase the positive value. In order to obtain a porous filter material having a positive zeta potential, various methods may be used. For example, there are a method for using a filter forming material having a positive zeta potential by itself; a method for forming a filter material after causing a substance for making zeta potential positive, for example, a substance having a cationic functional group, such as amino group, to be contained in or connected to the filter forming material; a method for forming a filter material from a mixture of a filter forming material and other material having a positive zeta potential; or a method for performing a treatment for causing zeta potential of filter material to be changed to positive after forming the filter material.

Specifically, there are a method for allowing a porous filter material made of a cellulose fiber or an organic polymer to react with a reactive (polymerizable) monomer having a positive zeta potential, such as a method for forming a filter material of cellulose fibers which have been allowed to react with a polyfunctional reactive quaternary ammonium to have a positive zeta potential, a method for forming a filter material by graft polymerizing a porous filter material of a porous membrane or fibers made of polypropylene with vinylpyridine by plasma polymerization, allowing the graft polymerized porous filter material to react with benzylchloride, and then quar-

ternizing amino group of pyridine, and a method for polymerizing monomer capable of applying a positive zeta potential, such as allyl amine hydrochloride salts, with a porous filter material made of an organic polymer; a method for directly forming a porous filter material of fiber, membrane, foam or other porous structures from a polymer or copolymer after producing the polymer or copolymer of polymerizable monomers having a cationic group; a method for treating a surface of a porous filter material by using the aforementioned polymer or copolymer having the cationic group; or a method for forming a porous filter material by binding glass fibers or glass particles with a binder having a positive zeta potential.

As the aforementioned quarternary ammonium compounds, there are 2-(meth)acryloyloxy alkyltrialkyl ammonium halides, such as 2-(meth)acryloyloxy ethyltrimethyl ammonium chloride, 2-(meth)acryloyloxy ehyltriethyl ammonium chloride, 2-(meth)acryloyloxy ethylpropyl ammonium chloride, 2-(meth)acryloyloxy ethyltrimethyl ammonium bromide, 2-(meth)acryloyloxy ethyltriethyl ammonium bromide, and a compound represented by the following formula II. This compound will be hereinafter referred to as "compound A".

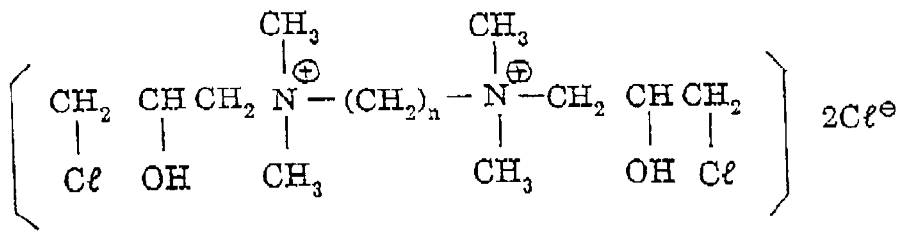

...(II)

It should be noted that a leukocyte-removing filter of this invention must have zeta potential of a positive value (including zero), i.e. a value of not less than 0 mV, preferably not less than 3 mV. If the zeta potential is less than zero, although leukocytes may be removed, removal ratio of thrombocytes is low. There is no specific limitation to the maximum value of zeta potential. However, in practice, it is difficult to obtain a porous filter material having zeta potential of not less than 25 mV.

Although there is no specific limitation to the shape and structure of an apparatus furnished with a leukocyte-removing filter of this invention, arid to a method for mounting or arranging the filter on the apparatus, the filter may be used in conventional types of apparatus. That is, the shape or the like of the apparatus may be suitable selected in accordance with the kind, material, shape, use of the porous filter material. A leukocyte-removing filter of this invention may comprise one or more porous filter layers. In a case where one porous filter layer is used, it has at least one surface on which zeta potential is a positive value in an electrolyte solution having a pH value of 5.0 to 7.0. In a case where a plurality of porous filter layers are used, at least one of the porous filter layers has a surface on which zeta potential is a positive value in an electrolyte solution having a pH value of 5.0 to 7.0. preferably, the filter layer having such a surface is arranged on the outlet side.

Referring now to the drawings, the preferred embodiments of a leukocyte-removing filter and a leukocyte-removing apparatus of this invention will be described below.

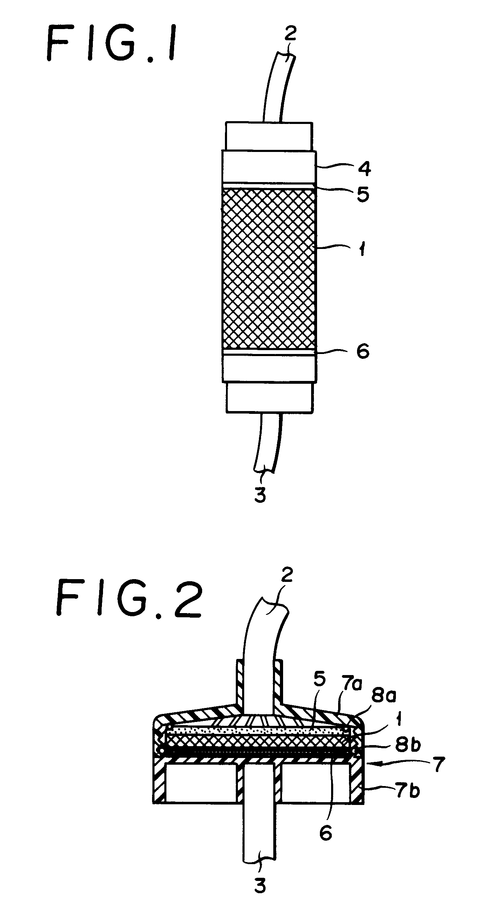

Fig. 1 shows the first preferred embodiment of a leukocyte-removing apparatus of this invention. In this embodiment, a porous filter material 1 (e.g. fibrous or granulated filter material) which has a positive zeta potential in an electrolyte solution having a pH value of 5.0 to 7.0, is housed within a cylindrical column 4 having a blood inlet 2 and a blood outlet 3. The porous filter material 1 is arranged between a prefilter 5 for preventing micro aggregated substances from being introduced into the porous filter material, and a mesh 6 for preventing the porous filter material from moving downwards.

Fig. 2 shows the second preferred embodiment of a leucocyte-removing apparatus of this invention. In this embodiment, a porous filter material 1 in the form of a sheet, film, membrane or plate, which material has a positive zeta potential in an electrolyte solution having a pH value of 5.0 to 7.0 is arranged in a holder 7 having a blood inlet 2 and a blood outlet 3 between the prefilter 5 made of synthetic resin, such as polypropylene and polyethylene, and the mesh 6. The holder 7 is made of synthetic resin, such as polypropylene, MBS, polymethyl methacrylate and carbonate, and it comprises an upper holder member 7a and a lower holder member 7b which engage with each other in the form of a screw via an O-ring 8b made of silicone rubber. The prefilter 5 is pressed downwards by a pressing portion of the upper holder member 7a via an O-ring 8a.

The effectiveness of a leukocyte-removing filter of this invention will be understood from the following Examples.

## Example 1

A solution was prepared by dissolving 510 g of a cationizing agent (trademark "Cationon UK" commercially

available from Npposha Yushi Kogyo, Co., Ltd.) and 77 g of sodium hydroxide in 3113 ml of distilled water. After 170 g of cellulose fibers (egyptian cotton) was dipped in this solution at 80 °C for 60 minutes, the cellulose fibers were removed from the solution and dried at 80 °C . Then, 2 g of the treated cellulose fibers were arranged in a flow potential measuring cell of Shimazu Flow Potential Measuring Apparatus ZP-10B, and flow potential thereof was measured at a temperature of 20± 5 °C by using 1 millimole KCl solution (pH 6± 1) as flow medium. As a result, the value Es/P (flow potential/pressure applied to the solution in order to cause the solution to flow) was 3.5 x $10^{-5}$. This value was used in the aforementioned formula I with coefficient of kinematic viscosity $\eta$ = 0.009142, dielectric constant $\varepsilon$ = 78.89 and electrical conductivity $\lambda$ = 1.31 x $10^{8}$ to obtain zeta potential of the cellulose fibers. As a result, its zeta potential was 6.7 mV.

The cylindrical column (inner diameter = 15.7 mm) of the leukocyte-removing apparatus of Fig. 1 was filled with 1.625 g of the aforementioned cellulose fibers along with the prefilter 5 having 0.8 mm of thickness and the mesh 6 so that the length of the filter layer was 42.0 mm and density of the cellulose fibers was 0.20 g/$cm^3$.

Then, 400 ml of human blood was collected in a blood bag (Tradenark "Terumo Double Bag" commercially available from Terumo Kabushiki Kaisha) while it was anti-coagulated with citrate-phosphate dextrose (CPD), and centrifugation of this human blood was performed at a rate of 3000 rpm for 10 minutes. Thereafter, 150 ml of blood plasma which is a supernatant was taken in a transfer bag. An erythrocytes suspension was prepared by adding about 150 ml of physiological saline solution to the remaining CRC so that hematocrit becomes 50%. Then, 25 ml of this suspension was introduced into the leukocyte-removing apparatus via the blood inlet 2, and the time required for the whole suspension to be discharged from the blood outlet 3 was measured. Furthermore, the numbers of leukocytes and thrombocytes were measured by a counter ("Sysmex NE-6000" commercially available from Toa Iyodenshi Co., Ltd.), and removal ratios of leukocytes and thrombocytes were calculated by the following formula.

(Number of Each Blood Cells Before Filtration - Number of Each Blood Cells After Filtration) x 100 / (Number of Each Blood Cells Before Filtration)

As a result, the required time was 2 minutes 40 seconds, the removal ratio of leukocytes was 96 %, and the removal ratio of thrombocytes was 97± 3 %.

As a reference, cellulose fibers (zeta potential = -23.9 mV) of the same types as that of the aforementioned embodiment were used without cationizing treatment, to determine the time required for the whole suspension liquid to be discharged from the blood outlet 3, and the removal ratios of leukocytes and thrombocytes in the same manner as that of the aforementioned embodiment. As a result, the required time was 3 minutes, the removal ratio of leukocytes was 95% and the removal ratio of thrombocytes was 58± 3%.

## Example 2

In 6 ml of an aqueous solution of saturated sodium hydroxide, 3 g of cellulose fibers and 15 ml of epichlorohydrin were add to allow a reaction at 50 °C for 2 hours. Then, the cellulose fibers were dipped in a methanol solution containing 0.2 moles of 4-aminopyridine to allow a reaction at 50 °C for 3 hours. Thereafter, the cellulose fibers were removed from the solution to be dipped in 500 ml of a methanol solution containing 2 moles of methyliodide to allow a reaction in a tightly sealed vessel at 30 °C for 24 hours. Zeta potential of the treated cellulose fibers was determined in the same manner as that of Example 1. As a result, it was + 9,2 mV.

Then, the cylindrical column as shown in Fig. 1 was filled with 1.625 g of the obtained cellulose fibers (with density of the fiber 0.20 g/$cm^3$), and the measurement was performed in the same manner as that of Example 1. As a result, the required time was 3 minutes 40 seconds, the removal ratio of leukocytes was 85 %, and the removal ratio of thrombocytes was 75.0 %.

As a reference, the same test was performed with respect to untreated cellulose fibers having -23.9 mV of zeta potential. As a result, the required time was 3 minutes 55 seconds, the removal ratio of leukocytes was 87.1 %, and the removal ratio of thrombocytes was 52.2 %.

## Example 3

In 100 ml of water, 1 g of a porous body (1.3 mm in thickness, about 10 $\mu$m in average pore diameter) of polyvinyl alcohol cross-linked with formaldehyde was dipped, and 2.5 ml of nitric acid solution containing 0.1 mol/liter cerium ammonium nitrate, which nitric acid solution was prepared by dissolving cerium ammonium nitrate in 1N nitric acid, was added thereto. In this solution, 5 g of acrylamide was further dissolved to allow polymerization under an atmosphere of nitrogen at 40 °C for 1 hour.

Zeta potential of the obtained porous body was determined in the same manner as that of Example 1. AS a result, it was +5.9 mV.

A disc of 25 mm diameter was punched out of this porous body. This disc was placed together with a prefilter

5 of 0.8 mm thickness and a mesh 6 in a holder shown in Fig. 2, and the same test as that of Example 1 was performed. As a result, the required time was 3 minutes 16 seconds, the removal ratio of leukocytes was 93.3 %, and the removal ratio of thrombocytes was 91.4 %.

As a reference, the same test was performed with respect to untreated porous body of polyvinyl alcohol cross-linked with formaldehyde (zeta potential = -22.8 mV). As a result, the required time was 2 minutes 29 seconds, the removal ratio of leukocytes was 92.0 %, and the removal ratio of thrombocytes was 65.5 %.

Furthermore, the pore size of porous body was measured by mercury intrusion method.

Example 4

A porous filter of polyurethane (0.5 mm in thickness, about 10 μm in average pore diameter) was washed with methanol in a Soxhlet apparatus for 8 hours, and then, it was irradiated with cold plasma (argon, 0.2 Torr) for 20 seconds. Subsequently, the porous filter together with 4-vinyl pyridine gas (0.5 Torr) were introduced into a reaction vessel, and graft polymerization of surface was performed. In a solution containing 0.1 M of benzyl chloride, the surface-grafted filter was quarternized at 55 °C for three hours. Thus, a porous filter of polyurethane having N-benzyl pyridinium chloride group on the surface thereof was obtained.

Zeta potential of this porous filter was determined in the same manner as that of Example 1, and it was +46.8 mV. Furthermore, the same test as that of Example 1 was performed. As a result, the required time was 4 minutes 50 seconds, the removal ratio of leukocytes was 99.89 %, and the removal ratio of thrombocytes was 98.84 %.

Example 5

In the same manner as that of Example 4, the same porous filter of polyurethane was graft polymerized, and quarternized in a methanol solution containing 0.1 M of ethyl bromide at 50 °C for 16 hours to produce a porous filter of polyurethane having a triethyl ammonium bromide group on the surface thereof.

Zeta potential of this porous filter was determined in the same manner as that of Example 1. As a result, the required time was 4 minutes 25 seconds, the removal ratio of leukocytes was 99.73 %, and the removal ratio of thrombocytes was 99.10 %.

As a reference, with respect to a untreated porous filter of polyurethane used as a substance in Examples 4 and 5, zeta potential was determined. As a result, it was -40.0 mV. Furthermore, this untreated porous filter of polyurethane was tested in the same manner as that of Example 1. As a results, the required time was 4 minutes 33 seconds, the removal ratio of leukocytes was 91.4 %, and the removal ratio of thrombocytes was 5.1 %.

Example 6

Two porous filters of polyurethane (0.5 mm in thickness, 25mm in diameter, about 15 μm in average pore diameter, zeta potential = -30 mV, removal ratio of leukocytes = about 80%) were prepared. One of these porous filters was graft polymerized with glycidylacrylate, and then, the same cationizing agent as that of Example 1 was fixed thereto. The zeta potential of the obtained porous filter was +7.9 mV. In the same manner of Example 3, the obtained porous filter was arranged on the blood outlet side in the holder of Fig. 1 to be used as a main filter, and another porous filter was arranged on the blood inlet side in the holder. CRC which contains 84.8 x $10^2$/μl of leukocytes and which has 73% of hematocrit value was filtered at a constant speed of 0.5 ml/min. $cm^2$ to determine the removal ratios of leukocytes and thrombocytes.

As a result, the removal ratio of leukocytes was 99.98%, and the removal ratio of thrombocytes was 95.38%. Furthermore, there was little blinding in the main filter after filtration for 15 minutes. In Examples 4, 5 and 6, the pore size was measured by the same method as that of Example 3.

From the aforementioned results, it was found that a leukocyte-removing filter and an apparatus furnished therewith, according to this invention, can effectively remove thrombocytes from a solution containing blood components without reducing pore size of the filter material.

## Claims

1. A filter for removing leukocytes and thrombocytes from a solution containing blood components, said filter comprising a filter material having a surface on which zeta potential is a positive value in an electrolyte solution having a pH value of 5.0 to 7.0.

2. A filter according to claim 1, wherein said zeta potential is not less than 3 mV.

3. A filter according to claim 1, wherein said surface is allowed to come into contact with said solution containing blood components.

4. A filter according to claim 1, wherein said filter material is made of cellulose fibers which has a pretreated surface so that zeta potential thereon is a positive value in an electrolyte solution having a pH value of 5.0 to 7.0.

5. A filter according to claim 1, wherein said filter material is a porous body made of polyvinyl alcohol cross-linked with formaldehyde, said porous body having a pretreated surface so that zeta potential thereon is a positive value in an electrolyte solution having a pH value of 5.0 to 7.0.

6. A filter according to claim 1, wherein said filter material is a porous body made of polyurethane, said porous body having a pretreated surface so that zeta potential thereon is a positive value in an electrolyte solution having a pH value of 5.0 to 7.0.

7. An apparatus for removing leukocytes and thrombocytes from a solution containing blood components, said apparatus comprising;
a filter having a surface on which zeta potential is a positive value in an electrolyte solution having a pH value of 5.0 to 7.0; and
a container for housing therein said filter, said container having an inlet for introducing said solution containing blood components into the interior thereof, and an outlet for discharging a filtrate to the outside.

8. An apparatus according to claim 7, wherein said filter comprises a plurality of filter layers, at least one of said filter layers having a surface on which zeta potential is a positive value in an electrolyte solution having a pH value of 5.0 to 7.0.

9. An apparatus according to claim 8, wherein said filter layer having said surface is arranged on the side of said outlet.

10. An apparatus according to claim 7, wherein said filter comprises two filter layers, at least one said filter layers having a surface on which zeta potential is a positive value in an electrolyte solution having a pH value of 5.0 to 7.0.

11. An apparatus according to claim 10, wherein said filter layer having said surface is arranged on the side of said outlet.

FIG.1

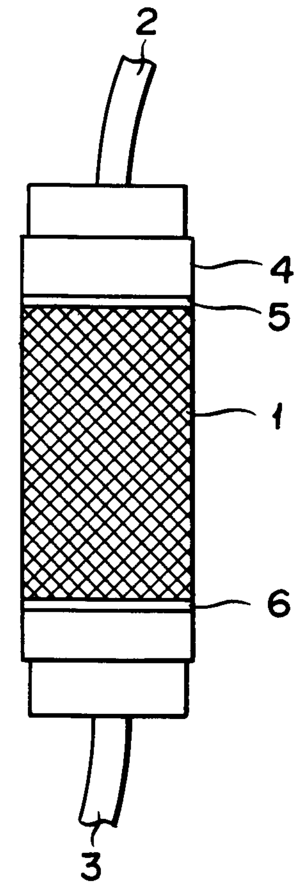


FIG.2